# EUROPEAN PATENT APPLICATION

(11) **EP 4 699 537 A1**
(43) Date of publication of application: **25.02.2026**
(21) Application number: 25192077.3
(22) Date of filing: 28.07.2025
(51) Int. Cl.: A61B 5/349, A61B 5/00, G16H 50/20

(54) **SYSTEMS AND METHODS FOR ECG INTERPRETATION BASED ON LONGITUDINAL CRITERIA**

(30) Priority: 22.08.2024 GR 20240100585; 27.08.2024 US 202418817090
(71) Applicant: GE Precision Healthcare LLC, Waukesha, WI 53188 (US)
(72) Inventor: KARGIANTOULAKIS, Emmanouil, Milwaukee, 53223 (US); SAKHONCHIK, Vladimir, Milwaukee, 53223 (US); ROWLANDSON, Gordon Ian, Milwaukee, 53223 (US); FARRELL, Robert, Milwaukee, 53223 (US)
(74) Representative: Kilburn & Strode LLP

(57) **Abstract**

Methods and systems for electrocardiogram (ECG) interpretation based on longitudinal medical data are here presented. In one example, a method, comprises, during a development phase of an ECG analysis model, generating a bank of longitudinal electrocardiogram (ECG) features from a plurality of ECGs with known diagnoses (306); extracting longitudinal criteria from the plurality of ECGs (410); during a deployment phase of the ECG analysis model, obtaining a plurality of ECGs of a patient (502, 506), wherein the plurality of ECGs includes a current ECG and one or more historical ECGs; determining, based on the longitudinal criteria, a diagnosis (510); and transmitting the diagnosis to a user device (514).

## Description

### CROSS REFERENCE TO RELATED APPLICATION

The present application claims priority to Greek Patent Application No. 20240100585, filed on August 22, 2024. The entire contents of the above-listed application is hereby incorporated by reference for all purposes.

### FIELD

Embodiments of the subject matter disclosed herein relate to longitudinal ECG parameters, and more particularly to systems and methods for generating longitudinal ECG parameters and determining diagnoses using the longitudinal ECG parameters.

### BACKGROUND

An ECG is a graphic representation of electrical activity of the heart, and is generally represented as a waveform. An ECG analysis model may receive an ECG, determine a set of features (e.g., findings) of the ECG, compare the set of features to respective criteria of a set of rules, determine a diagnosis based on the criteria met by the features of the ECG, and generate a diagnostic interpretation result of the ECG. A physician may review the ECG and/or the diagnostic interpretation result to assess the cardiac activity of a patient.

A rule or set of rules of the ECG analysis model may correspond to a particular diagnosis. Further, a rule may include various criteria that, if satisfied, cause the ECG analysis model to determine the particular diagnosis. A criterion may correspond to a particular feature of the ECG, and may include a relevant threshold. As an example, ECG analysis model may use a criterion corresponding to a feature of a QRS duration being greater than or equal to 120 ms. The criterion for a QRS duration greater than 120 ms may be a requirement/precondition for the ECG analysis model to determine a particular diagnosis, such as left bundle branch block (LBBB). However, other diagnoses, such as left ventricular hypertrophy (LVH) may have similar ECG patterns and satisfy similar criteria by the ECG model. Differentiating between a more impactful/sever diagnosis (e.g., like LBBB) from its mimics (e.g., like LVH for LBBB) is challenging for any ECG analysis system.

### BRIEF DESCRIPTION

In one example, a method comprises, during a development phase of an ECG analysis model, generating a bank of longitudinal electrocardiogram (ECG) features from a plurality of ECGs with known diagnoses; extracting longitudinal criteria from the plurality of ECGs; during a deployment phase of the ECG analysis model, obtaining a plurality of ECGs of a patient, wherein the plurality of ECGs includes a current ECG and one or more historical ECGs; determining, based on the longitudinal criteria, a diagnosis; and transmitting the diagnosis to a user device.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, wherein below:
FIG. 1 is a diagram of an example system for electrocardiogram (ECG) interpretation based on longitudinal parameters;
FIG. 2 is a diagram of example components of a device of the example system shown in FIG. 1;
FIG. 3 is a flowchart illustrating a method for generating longitudinal features;
FIG. 4 is a flowchart illustrating a method for determining a diagnosis for an ECG using longitudinal features;
FIG. 6 is a flowchart illustrating a method for extracting, using an AI model, criteria for determining a diagnosis by a ECG analysis model;
FIG. 7 is an example graph of an ECG parameter for a first diagnosis;
FIG. 8 is another example graph of the ECG parameter of FIG. 7 for a second diagnosis; and
FIG. 9 is an example user interface of patient records.

### DETAILED DESCRIPTION

The following description relates to various embodiments of electrocardiogram (ECG) interpretation based on longitudinal features. In particular, systems and methods for generating longitudinal features and interpreting an ECG based on those longitudinal features are provided.

ECG analysis models can be used to determine a diagnosis for an ECG based on features and criteria identified within the ECG. For example, the ECG analysis model may be configured with sets of rules that define sets of criteria (e.g., one or more features that together can be satisfied). The ECG analysis model may ingest the ECG data to determine which criteria the ECG satisfies and based thereon, output a diagnosis. In general, ECG analysis models reduce time spent by clinicians, experts, and other users in manually analyzing ECGs.

However, ECG analysis model based ECG interpretation is usually performed for features and criteria of single ECG. For example, features of the single ECG are fed into the ECG analysis model and a diagnosis is outputted. Analysis of a single ECG does not account for the progression of certain criteria over time. For example, trends of certain criteria may provide clinical significance for diagnosis determination. As a non-limiting example, criteria for a diagnosis of left bundle branch block (LBBB) and criteria for a diagnosis for left ventricular hypertrophy (LVH) may be similar within a single ECG. On the contrary though, trends of criteria over time may differ for LBBB vs LVH.

In practice, a subject-matter expert (e.g., a physician, ECG technician, or the like) may compare a current diagnosis to one or more previous ECGs for the patient to determine a diagnosis in circumstances where longitudinal data proves useful. However, manual comparison may be time consuming and subject to human error. For example, an ECG parameter of QRS duration may be analyzed by the expert. In LBBB, QRS duration typically increases suddenly while in LVH, QRS increases gradually over time. However, for a patient with LBBB, the expert may not look back far enough into the patient's record to find an ECG prior to a sudden jump in QRS duration, leading to the conclusion that the QRS duration increased gradually and thus an erroneous diagnosis of LVH may be determined by the expert. Further, the expert may not have the time and/or easy access into the longitudinal record in order to examine the evolution of relevant parameters. Therefore, without access to relevant differentiating information between more than one plausible diagnosis or the time to properly examine differentiating information that may come from longitudinal ECG data, an erroneous diagnosis is more likely.

Thus, systems and methods for ECG interpretation based on longitudinal ECG features are herein presented. Longitudinal features, as herein described, may include ECG features that are based in comparison of at least two ECGs. For example, a difference between a value of an ECG parameter in a first and a second ECG may be a first kind of longitudinal feature, a difference between a value of an ECG parameter and an average of all values of that ECG parameter for a given patient may be a second kind of longitudinal feature, and so on. Longitudinal criteria may include combinations of one or more longitudinal ECG features that can be satisfied by patient data (e.g., a current or most recent ECG and one or more historical ECGs of a patient). Longitudinal criteria may correspond to different diagnoses such that satisfaction of a particular longitudinal criteria yields an output of a corresponding diagnosis.

By generating one or more longitudinal ECG features, ECGs of patients may be interpreted, for example by an ECG analysis model, based on criteria that are extracted from both longitudinal and single-ECG features. Thus, ECG interpretation and diagnosis output via a rule-based model may be more accurate by accounting for longitudinal data of the patient.

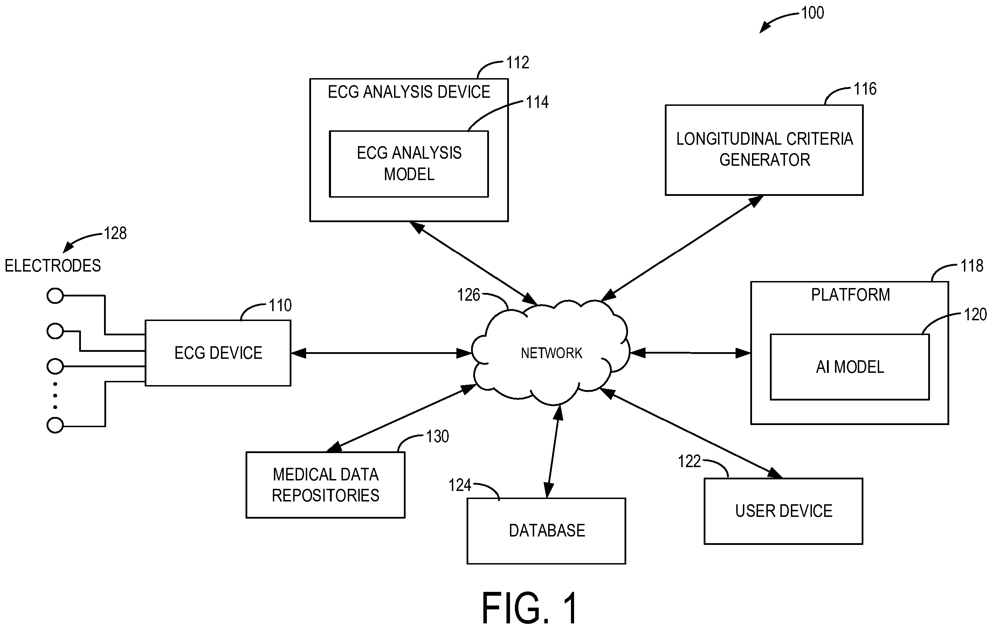

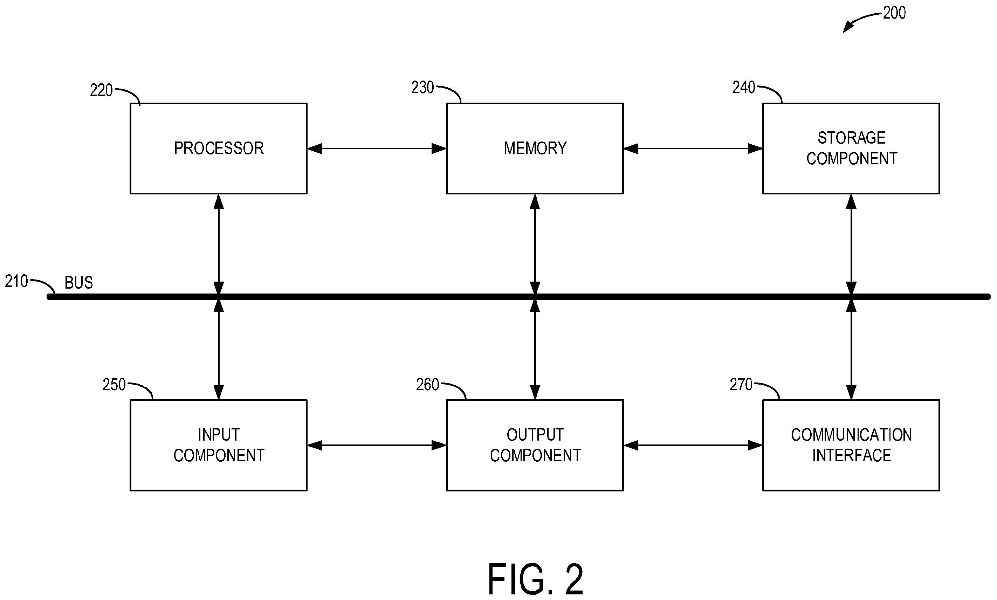

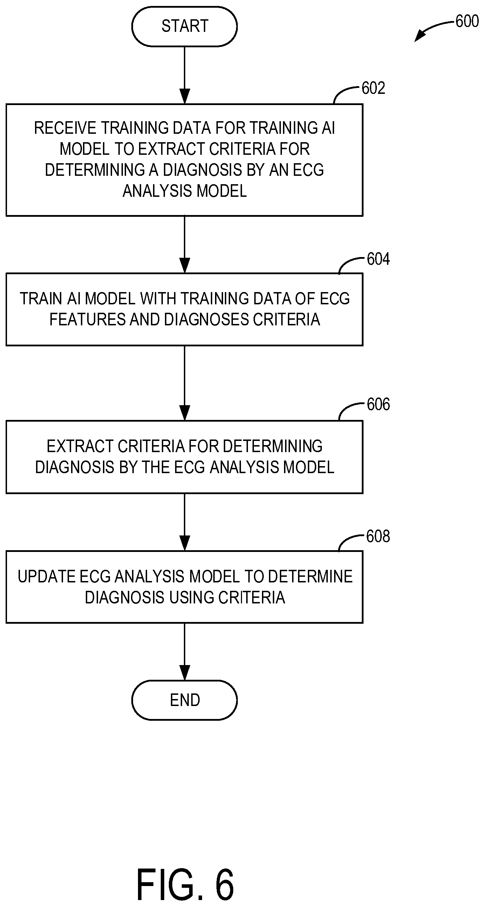

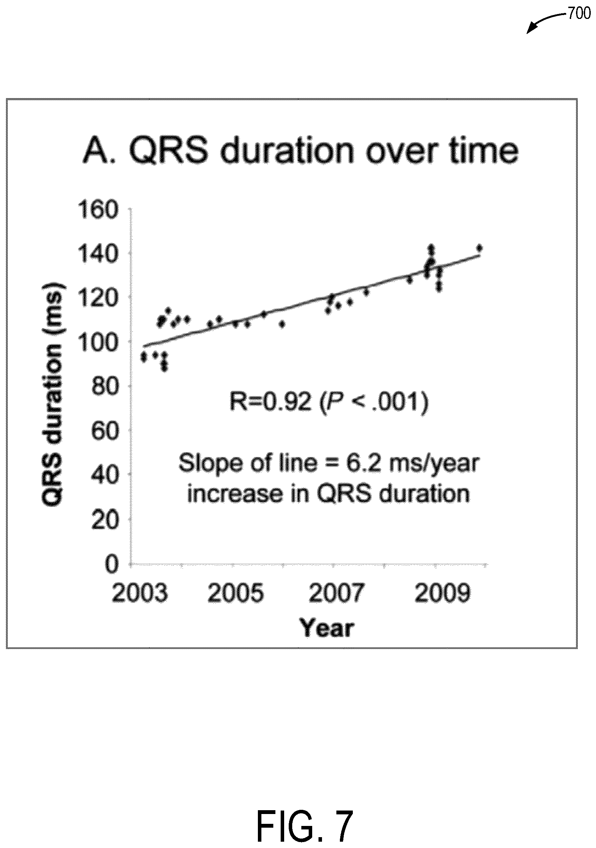

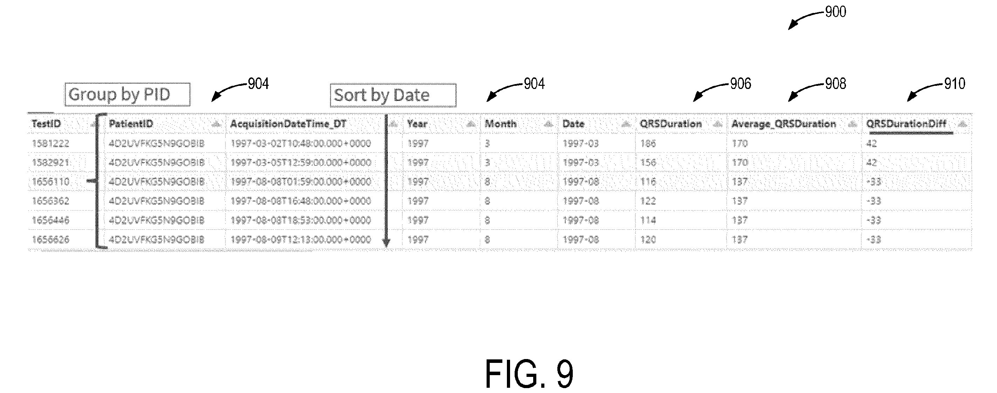

The systems and methods herein disclosed will now be described, by way of example, with reference to the figures, wherein FIG. 1 shows a diagram of an example ECG analysis system, FIG. 2 shows a diagram of example components of a device of the example system shown in FIG. 2, FIGS. 3-5 show flowcharts illustrating methods for generating longitudinal features, interpreting ECGs based on longitudinal features, and extracting criteria for determining a diagnosis by a ECG analysis model, FIGS. 6 and 7 show graphs of an example ECG parameter for different diagnoses over time; and FIG. 9 shows an example user interface of patient records.

Starting with FIG. 1, a diagram of an example ECG analysis system 100 is shown. The ECG analysis system 100 may be configured to extract interpretable criteria and generate longitudinal criteria for determining a diagnosis by an ECG analysis model. As shown in FIG. 1, the system 100 may include an ECG device 110, a plurality of electrodes 128, an ECG analysis device 112, an ECG analysis model 114, a longitudinal criteria generator 116, a platform 118, an AI model 120, a user device 122, a database 124, one or more medical data repositories 130, and a network 126.

ECG device 110 may be configured to generate an ECG tracing of a patient. For example, the ECG device 110 may be a stand-alone ECG device, a portable ECG device, a multi-vital sign monitoring device, or the like. The ECG device may receive cardiac electrical signals via the plurality of electrodes 128, and may generate the ECG tracing based on the cardiac electrical signals. The ECG may be a single-lead ECG, a 3-lead ECG, a 5-lead ECG, a 6-ead ECG, a 12-lead ECT, or the like. The ECG device 110 may include any number of electrodes 128. For example, the ECG device 110 may include electrodes for generating a 12-lead ECG. In this example, the leads may include leads I, II, III, aVF, aVR, aVL, V1, V2, V3, V4, V5, and V6. The ECG device 110 may be configured to use a subset of the standard 12 leads, or alternatively an ECG using non-standard lead placements (e.g., such as Holter monitor lead placements) or synthesized ECG leads using the actual acquired lead set (e.g., such as GE HealthCare's 12RL algorithm used to synthesize a 12 lead ECG from a reduced lead set).

The ECG analysis device 112 may be configured to receive the ECG from the ECG device 110, determine a set of features of the ECG using the ECG analysis model 114, compare the set of features to respective criteria of a set of rules using the ECG analysis model 114, determine a diagnosis using the ECG analysis model 114, generate a diagnostic interpretation result of the ECG using the ECG analysis model 114, and transmit the diagnostic interpretation result to another device, to a display, or the like.

The ECG analysis model 114 may be a rules-based model, a machine learning model (e.g., a deep neural network, a convolutional neural network, or the like), a decision tree, or other type of model configured to ingest ECG data and output a diagnosis. For example, when the ECG analysis model 114 is a rules-based model, the ECG analysis model 114 may be configured to receive the ECG from the ECG device 110, receive one or more previous ECGs of the patient from the one or more medical data repositories 130, determine a set of features of the ECG (e.g., single-ECG features) and longitudinal features of the ECG and one or more previous ECGs, compare the set of single-ECG features and longitudinal features to respective criteria (e.g., single-ECG criteria and longitudinal criteria) of a set of rules, determine a diagnosis, and generate a diagnostic interpretation of the ECG. For example, the ECG analysis model 114 may be the Marquette^{™} 12SL ECG analysis program, or the like.

The longitudinal criteria generator 116 may be configured to generate, based on population data (including diagnoses and ECG findings), single patient data (including diagnoses and ECG findings), and subject-matter expert inputs, a bank of longitudinal criteria and corresponding diagnoses. The generated longitudinal criteria and corresponding diagnoses may be fed back to the ECG analysis model 114 to be stored for comparison with acquired patient data (e.g., a current ECG and one or more previous ECGs).

The platform 118 may be configured to extract, using the AI model 120, interpretable criteria for determining a diagnosis by the ECG analysis model 114. For example, the platform 118 may be a server, a cloud computing system, or the like. The interpretable criteria that are extracted may include one or both of single-ECG criteria and longitudinal criteria.

The AI model 120 may be configured to extract interpretable criteria for determining a diagnosis by the ECG analysis model 114. As noted, the interpretable criteria may include one or both of single-ECG criteria and longitudinal criteria. For example, the AI model 120 may be a decision tree (e.g., a classification tree, a regression tree, or the like), a linear regression model, a neural network (e.g., a deep neural network (DNN), a convolutional neural network (CNN), a recurrent neural network (RNN), or the like), a logistic regression model, a support vector machine, or the like.

The user device 122 may be configured to receive the criteria for determining a diagnosis by the ECG analysis model 114, and provide the criteria for determining a diagnosis by the ECG analysis model 114 for display. For example, the user device 122 may be a smartphone, a laptop computer, a desktop computer, a wearable device, a medical device, or the like.

The database 124 may be configured to store an ECG, a set of features of the ECG, a diagnosis of the ECG, a diagnostic interpretation of the ECG, modification information, patient information associated with the ECG, a classification target of the ECG, or the like. Further, the database 124 may be configured to store criteria for determining a diagnosis by the ECG analysis model 114, including single-ECG criteria and/or longitudinal criteria. For example, the database 124 may be a hierarchical database, a network database, a relational database, or the like. Criteria stored in the database 124 may be initially generated by a subject-matter expert, in some examples. For example, the database 124 may be a database of the Marquette^{™} 12SL ECG analysis program, or other similar program.

The one or more medical data repositories 130 may include one or more of an electronic medical record (EMR) database, an ECG database, a radiology information system (RIS), a picture archiving and communication system (PACS), or other typed of database configured to store medical records of a plurality of patients. For example, ECGs performed for a patient may be stored in an ECG database with associated data including single-ECG features, corresponding diagnostic interpretation, a date of acquisition, an ordering provider, and the like.

The network 126 may be configured to permit communication between the devices of the system 100. For example, the network 126 may be a cellular network (e.g., a fifth generation (5G) network, a long-term evolution (LTE) network, a third generation (3G) network, a code division multiple access (CDMA) network, etc.), a public land mobile network (PLMN), a local area network (LAN), a wide area network (WAN), a metropolitan area network (MAN), a telephone network (e.g., the Public Switched Telephone Network PSTN)), a private network, an ad hoc network, an intranet, the Internet, a fiber optic-based network, or the like, and/or a combination of these or other types of networks.

As an example, the longitudinal criteria generator 116 may communicate with the ECG analysis device 112 to transmit generated longitudinal features over the network 126. Further, the ECG analysis device 112 may access data from the database 124 and/or the one or more medical data repositories 130 over the network 126. In this way, the system 100 may be an interconnected system by which the modules thereof communicate with each other to execute various processes and/or methods.

The number and arrangement of the devices of the system 100 shown in FIG. 1 are provided as an example. In practice, the system 100 may include additional devices, fewer devices, different devices, or differently arranged devices than those shown in FIG. 1. Additionally or alternatively, a set of devices (e.g., one or more devices) of the system 100 may perform one or more functions described as being performed by another set of devices of the system 100.

FIG. 2 shows a diagram of example components of a device 200 of the example ECG analysis system 100 shown in FIG. 1. The device 200 may correspond to the ECG device 110, the ECG analysis device 112, the platform 118, the user device 122, the longitudinal criteria generator 116, and/or the database 124. As shown in FIG. 2, the device 200 may include a bus 210, a processor 220, a memory 230, a storage component 240, an input component 250, an output component 260, and a communication interface 270.

The bus 210 may include a component that permits communication among the components of the device 200. The processor 220 may be implemented in hardware, firmware, or a combination of hardware and software. The processor 220 may be a central processing unit (CPU), a graphics processing unit (GPU), an accelerated processing unit (APU), a microprocessor, a microcontroller, a digital signal processor (DSP), a field-programmable gate array (FPGA), an application-specific integrated circuit (ASIC), or another type of processing component. The processor 220 may include one or more processors capable of being programmed to perform a function. Specifically, the processor 220 may include one or more processors 220 configured to perform the operations described herein. Alternatively, multiple processors 220, collectively, may be configured to perform the operations described herein, and each of the multiple processors 220 may be configured to perform a subset of the operations described herein. For example, a first processor 220 may perform a first subset of the operations described herein, a second processors 220 may be configured to perform a second subset of the operations described herein, etc.

The memory 230 may include non-transitory memory, random access memory (RAM), a read only memory (ROM), and/or another type of dynamic or static storage device (e.g., a flash memory, a magnetic memory, and/or an optical memory) that stores information and/or instructions for use by the processor 220. For example, the memory 230 may store executable instructions thereon that are executable by the processor 220 to perform the operation described herein.

The storage component 240 may store information and/or software related to the operation and use of the device 200. For example, the storage component 240 may include a hard disk (e.g., a magnetic disk, an optical disk, a magneto-optic disk, and/or a solid state disk), a compact disc (CD), a digital versatile disc (DVD), a floppy disk, a cartridge, a magnetic tape, and/or another type of non-transitory computer-readable medium, along with a corresponding drive.

The input component 250 may include a component that permits the device 200 to receive information, such as via user input (e.g., a touch screen display, a keyboard, a keypad, a mouse, a button, a switch, a camera, and/or a microphone for receiving the reference audio input and/or visual input). Additionally or alternatively, the input component 250 may include a sensor for sensing information (e.g., a global positioning system (GPS) component, an accelerometer, a gyroscope, and/or an actuator). The output component 260 may include a component that provides output information from the device 200 (e.g., a display, a speaker for outputting sound at the output sound level, and/or one or more light emitting diodes (LEDs)).

The communication interface 270 may include a transceiver-like component (e.g., a transceiver and/or a separate receiver and transmitter) that enables the device 200 to communicate with other devices, such as via a wired connection, a wireless connection, or a combination of wired and wireless connections. The communication interface 270 may permit the device 200 to receive information from another device and/or transmit information to another device. For example, the communication interface 270 may include an Ethernet interface, an optical interface, a coaxial interface, an infrared interface, a radio frequency (RF) interface, a universal serial bus (USB) interface, a Wi-Fi interface, a cellular network interface, or the like.

The device 200 may perform one or more processors and methods herein described. The device 200 may perform these processes and/or methods based on the processor 220 executing software instructions stored in a non-transitory computer-readable medium, such as the memory 230 and/or the storage component 240. A computer-readable medium may be defined herein as a non-transitory memory device. A memory device may include memory space within a single physical storage device or memory space spread across multiple physical storage devices.

The software instructions may be read into the memory 230 and/or the storage component 240 from another computer-readable medium or from another device via the communication interface 270. When executed, the software instructions stored in the memory 230 and/or the storage component 240 may cause the processor 220 to perform one or more processes and/or methods described herein. Additionally or alternatively, hardwired circuitry may be used in place of or in combination with software instructions to perform one or more methods and/or processes described herein. Thus, implementations described herein are not limited to any specific combination of hardware circuitry and software.

The number and arrangement of the components shown in FIG.2 are provided as an example. In practice, the device 200 may include additional components, fewer components, different components, or differently arranged components than those shown in FIG. 2. Additionally or alternatively, a set of components (e.g., one or more components) of the device 200 may perform one or more functions described as being performed by another set of components of the device 200.

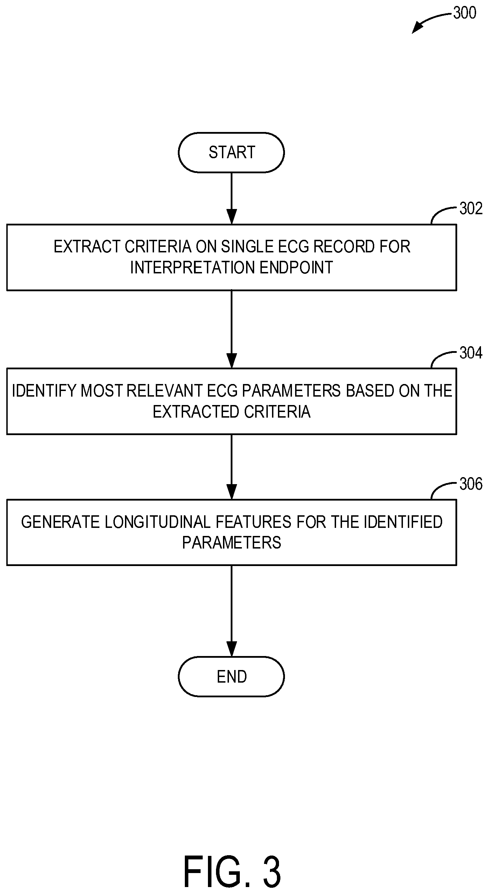

Referring now to FIG. 3, a flowchart illustrating a method 300 for generating longitudinal features is shown. The method 300 may be executed by one or more processors (e.g., processor 220) according to instructions stored in non-transitory memory (e.g., memory 230).

At 302, method 300 includes extracting criteria on a single ECG record for an interpretation endpoint. As an example, the single ECG record may be one of a plurality of ECG records in an ECG dataset for which diagnoses are known. An exemplary method for extracting criteria is described further with respect to FIG. 6. The criteria extracted may include combinations of ECG features (e.g., sums and differences of a parameters across different leads, or products and ratios of different parameters within the same lead) and/or sub-combinations of ECG features that together lead to the interpretation endpoint. The interpretation endpoint may be a diagnostic interpretation, a diagnosis, or the like for which the criteria are used to output.

At 304, method 300 includes identifying one or more relevant ECG parameters based on the extracted criteria. As an example, the single ECG may comprise a plurality of features, parameters, or findings. For example, parameters may include an amplitude of a P wave, a duration of the P wave, an amplitude of a Q wave, a duration of the Q wave, an amplitude of an R wave, a duration of the R wave, a PR interval, an amplitude of an S wave, a duration of the S wave, a duration of the QRS complex, an amplitude of a T wave, a duration of the T wave, a QT interval, or the like. Additionally or alternatively, the set of features may be defined by the data from a single lead (e.g., single lead specific P wave amplitude or P wave duration), or by data from multiple leads (e.g., global QT duration from earliest Q onset to latest T offset across all leads, or QT dispersion, which is a difference between shortest and longest single lead QT interval measurement across all leads), or by data from spatial relationships across multiple leads (e.g., spatial QRS-T angle or ventricular gradient). Based on the interpretation endpoint, the most relevant parameters may be identified. For example, if an interpretation endpoint is a diagnosis of LBBB, the most relevant parameters may include QT interval, duration of QRS complex, relation of R wave to S wave in lead V1, identified rhythm (e.g., super-ventricular), and the like.

At 306, method 300 includes generating longitudinal features for the identified parameters. The single ECG for which the relevant ECG parameters are identified may also have one or more ECGs previously acquired on the same patient. The longitudinal features may capture history of the identified parameters for the given patient. As an example, for an identified parameter of QRS complex duration, a longitudinal feature that is generated may be a QRS duration difference (QRSDD) between the current ECG (e.g., the considered single ECG) and an immediate previous ECG or another selected previous ECG, and/or a QRSDD normalized for a given time period. The longitudinal features for the QRS complex duration may thus indicate trends of QRS duration over time, with respect to a difference in the parameter compared to a given previous value, a difference compared to a mean, a change in trend (e.g., if a first trend is observed for a first period of time and a second, different trend is observed for a second period of time, as may be seen in diagnoses that cause a sudden effect on a parameter).

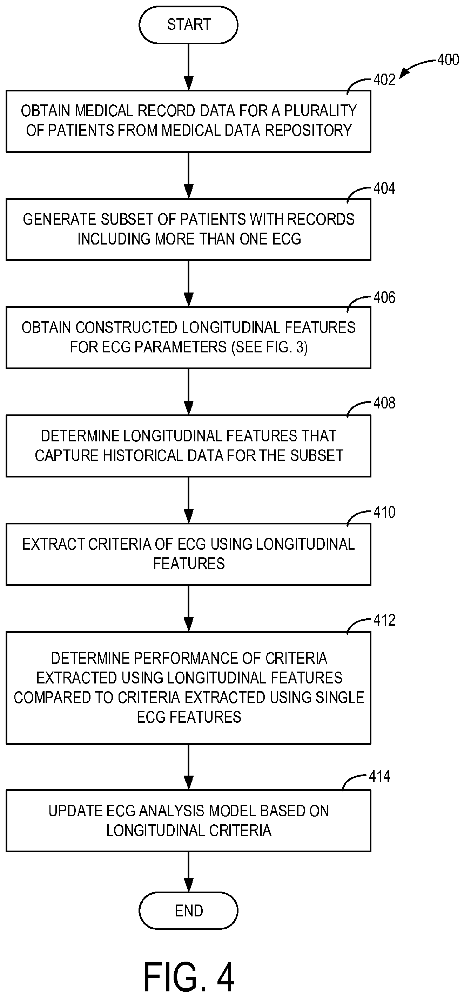

In some examples, method 300 may be repeated for a plurality of single ECG records, for example for a variety of different interpretation endpoints. In this way, a bank of longitudinal features that correspond to different interpretation endpoints may be created. The bank of longitudinal features may be stored in memory and in some examples may be fed back to the ECG analysis model in order for the model to use the longitudinal features to determine a diagnosis of a newly acquired ECG, as will be described herein with respect to FIG. 4. In this way, by generation of longitudinal features, ECGs may be analyzed for not only features of the ECG itself, but also with respect to historical ECGs, thereby saving time for the provider in having to compare ECGs manually, as well as increasing accuracy of diagnostic outputs.

Turning now to FIG. 4, a flowchart illustrating a method 400 for determining a diagnosis using longitudinal ECG features. The longitudinal ECG features may be constructed as described with respect to method 300 of FIG. 3. The method 400 may be executed by one or more processors (e.g., processor 220) according to instructions stored in non-transitory memory (e.g., memory 230). In some examples, the method 400 may be carried out according to an ECG analysis model, such as ECG analysis model 114 of FIG. 1. The method 400 may be executed as part of a development phase of the ECG analysis model.

At 402, method 400 includes obtaining medical record data for a plurality of patients from a medical data repository. For example, the ECG analysis model 114 of FIG. 1 may obtain data from one or more of the one or more medical data repositories. For example, the ECG analysis model 114 may obtain data from an ECG database that stores ECGs obtained for a plurality of patients with corresponding information including date of acquisition, diagnostic interpretation, chief complaint, and the like.

At 404, method 400 includes generating a subset of patients with records including more than one ECG based on the obtained medical record data. For example, the medical record data may include data of a plurality of patients. Some of the plurality of patients may have had only one ECG performed while others of the plurality of patients may have had more than one ECGs performed, wherein the medical record data that is obtained comprises more than one ECG assigned to each of those patients. The subset of patients with records including more than one ECG may thus include patients and corresponding ECGs for each of those patients.

At 406, method 400 includes obtaining constructed longitudinal features for ECG parameters. As described with respect to FIG. 3, a bank of longitudinal features may be constructed from a plurality of ECGs with various interpretation endpoints (e.g., known diagnoses). For example, longitudinal features corresponding to identified relevant parameters of a plurality of single ECGs with a given interpretation endpoints may be generated. Longitudinal features may thus be generated for a plurality of interpretation endpoints, thus constructing a bank of longitudinal features.

At 408, method 400 includes determining longitudinal features that capture the historical data for the subset. As an example, the bank of longitudinal features may comprise longitudinal features for a variety of ECG parameters and a variety of interpretation endpoints. The subset of patients and ECGs may comprise a subset of the variety of ECG parameters. Thus, irrelevant longitudinal features, based on the ECG parameters within the subset that are to be analyzed, may be filtered out, thereby reducing demanded processing power for ECG analysis.

At 410, method 400 includes extracting criteria of a selected ECG using the longitudinal features determined to be relevant for the subset. The selected ECG may have corresponding previous ECGs known for it. For example, the selected ECG may be a most recently performed ECG for a selected patient. The most recently performed ECG may have one or more corresponding previous ECGs to which it corresponds. The one or more corresponding previous ECGs may have known dates of acquisition and features therein as well as known diagnoses. The criteria may be extracted based on the features identified within the selected ECG and the diagnosis thereof and the corresponding one or more previous ECGs thereof. An exemplary method for extracting criteria is given in FIG. 6.

As noted, the method 400 may be executed during the development phase of the ECG analysis model. As such, extracting criteria may be performed on a retrospective database of ECGs for which the correct diagnosis of each ECG is known. Thus, the criteria are extracted for interpretation endpoints (e.g., target) of the known diagnoses.

In some examples, criteria of the selected ECG may be extracted for single-ECG features rather than longitudinal features as well. For example, similar to as described for the method for generation of the bank of longitudinal features, single-ECG criteria may be extracted (e.g., similar to at 302 of method 300). In such examples, at 412, method 400 includes determining a performance of criteria extracted using longitudinal features compared to criteria extracted using single-ECG features. Thus, as will be described with respect to FIG. 6, when the performance of criteria extracted using longitudinal features is higher, the longitudinal criteria may be used when generating a diagnostic output from the ECG analysis model during a deployment phase of the model. This determination of performance refers to establishing which set of criteria is better, based on analysis on a retrospective database. For example, the retrospective database of ECGs holds the ground truth for the correct interpretation of each ECG, and therefore the performance can be assessed therein. This determination may be performed in the development phase and is not repeated again during deployment, therefore there is no dependence on a newly acquired ECG (acquired for interpretation by the ECG analysis model) and no demand for a connection to the retrospective database during deployment of the model.

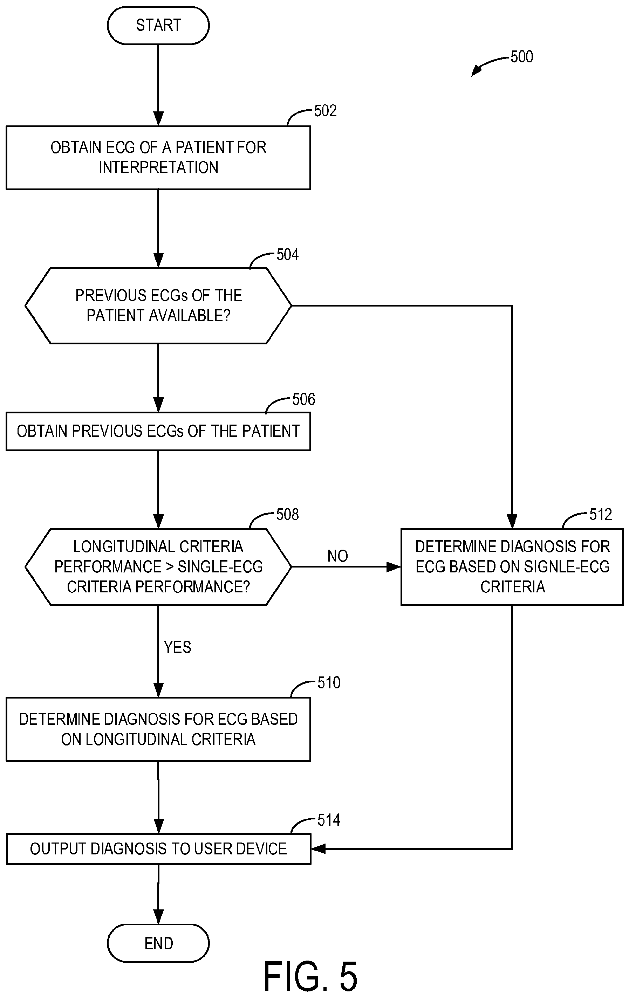

At 414, method 400 includes updating the ECG analysis model based on the longitudinal criteria. For example, the ECG analysis model may be updated or otherwise configured to determine diagnoses of newly acquired or obtained ECGs based on one or both of the longitudinal criteria and single-ECG criteria. Thus, based on a bank of longitudinal features generated from a retrospective database of single ECGs, as described with respect to FIG. 3, a set of longitudinal criteria may be defined, as herein described. The set of longitudinal criteria may be a fixed set that may then later be used for determination of a diagnosis for a newly acquired ECG when the ECG analysis model is deployed, as will be described with respect to FIG. 5.

Turning now to FIG. 5, a method 500 for determining a diagnosis of an ECG based on longitudinal criteria is shown. The method 500 may be executed by one or more processors of a device based on instructions stored in memory of the device. For example, the method 500 may be executed by processors based on instructions of an ECG analysis model (e.g., ECG analysis model 114) configured to determine a diagnosis of an ECG based on one or both of single-ECG criteria and longitudinal criteria. As explained with respect to FIGS. 3 and 4, the longitudinal criteria may be extracted based on a bank of generated longitudinal features from a set of single-ECGs, for which a diagnosis is known, which have associated previous ECGs. Method 500 as herein presented may be a deployment phase of the ECG analysis model, whereby the ECG analysis model is developed based on the methods of FIGS. 3 and 4.

At 502, method 500 includes receiving an ECG of a patient for interpretation. The ECG device may be a newly acquired ECG, obtained from an ECG device or from an ECG database. For example, as described with respect to FIG. 1, the ECG analysis model may be in communication with the ECG device (e.g., ECG device 110). The ECG may be acquired by the ECG device and transmitted directly to the ECG analysis device that includes the ECG analysis model. In other examples, the ECG analysis model may be in communication with an ECG database, for example an ECG database of the one or more medical data repositories 130 of FIG. 1, and the ECG may be obtained from the ECG database rather than directly from the ECG device.

At 504, method includes determining if one or more previous ECGs of the patient are available. For example, one or more previously acquired ECGs may be stored in the ECG database (e.g., of the one or more medical data repositories 130). The one or more previously acquired ECGs may be associated with the patient based on one or more patient demographics, such as name, date, and/or medical record number. For example, a patient record associated with the patient may comprise one or more previous ECGs available therein. If previous ECGs are available for the patient, method 500 proceeds to 506. If no previous ECGs are available for the patient, method 500 proceeds to 510.

At 506, method 500 includes obtaining the one or more previous ECGs of the patient. As noted, the one or more previous ECGs may be stored in an ECG database or other medical data repository to which the ECG analysis device is in communication (e.g., via a network). The ECG analysis device may retrieve the one or more previous ECGs from the ECG database or other medical data repository in response to determination that the one or more previous ECGs are available. In some examples, the ECG (e.g., a most current ECG) and the one or more previous ECGs may be retrieved at substantially the same time, for example when the ECG and the one or more previous ECGs are stored within the same database. In other examples, the current ECG may be obtained first, for example directly from the ECG device, and the one or more previous ECGs may be obtained separately from the medical data repository.

At 508, method 500 determines whether the performance of the longitudinal criteria is greater than the performance of the single-ECG criteria. As described with respect to method 400, when the longitudinal criteria are extracted based on the generated longitudinal features of ECGs for given interpretation endpoints, the performance of the longitudinal criteria may be assessed. In some examples, the performance may be compared to the performance of single-ECG criteria for the same set of ECGs and interpretation endpoints. If the performance of the longitudinal criteria is greater, method 500 proceeds to 510. If the performance of the single-ECG criteria is greater, method 500 proceeds to 512.

At 512, method 500 includes determining a diagnosis of the obtained ECG (e.g., the newly acquired ECG) based on single-ECG features. As noted, if the performance of the single-ECG criteria is greater than the performance of the longitudinal criteria, the ECG analysis model may be deployed based on single-ECG criteria. For example, the single-ECG criteria may be extracted for a retrospective ECG dataset. As noted, an exemplary extraction method is provided in FIG. 6. The diagnosis of the ECG may then be determined by the ECG analysis model according to those single-ECG criteria that are extracted.

At 510, when the performance of the longitudinal criteria is higher than the single-ECG criteria, method 500 includes determining a diagnosis for the ECG based on the longitudinal criteria. As noted, the ECG analysis model may be configured to determine a diagnosis based on ECG features and extracted criteria. In this example, the ECG analysis model may be configured to determine the diagnosis based on features of the ECG and corresponding previous ECG(s) and extracted longitudinal criteria. To clarify, features may be individual findings of ECG parameters within single ECGs and/or longitudinal features that indicate trends or other historical ECG data (e.g. QT interval, QRS complex duration, QRSDD, etc.), criteria may be sets of features for example a first feature and a second feature together may be a criterion but the first feature alone may not satisfy the criterion. Thus, the longitudinal features identified within the ECG and one or more corresponding previous ECGs may satisfy one or more of the fixed longitudinal criteria that were extracted during model development in order to determine the diagnosis. As such, the diagnosis may be a diagnosis that is indicated based on satisfied criteria based on the longitudinal features.

At 514, method 500 includes outputting the diagnosis, whether determined based on longitudinal criteria or single-ECG criteria, to a user device. For example, the diagnosis may be outputted for display on the user device (e.g., user device 122). Thus, the provider may review the outputted diagnosis as well as the longitudinal features, met criteria, and diagnostic interpretation together to accept or reject the outputted diagnosis.

Diagnoses determined based at least in part on longitudinal features may thus account for historical and temporal medical data that otherwise may be neglected. As a non-limiting example, the ECG parameter of QRS complex duration is a main diagnostic parameter for both LBBB and LVH. For a single ECG, a QRS duration above 120 may indicate either of LBBB or LVH. However, it is difficult to ascertain by other ECG features which diagnosis to output. Longitudinal features, however, may provide greater insight for distinguishing the two diagnoses.

Turning briefly to FIG. 7, a graph 700 is shown. The graph 700 depicts QRS duration over time in a patient with LVH. For example, a patient may undergo multiple ECGs over a period of multiple years. Each of those multiple ECGs may include a determined QRS duration. The QRS duration is plotted over time. As shown, in LVH, QRS duration gradually increases over time. For example, an average increase of 6.2 ms/year is depicted in the graph 700.

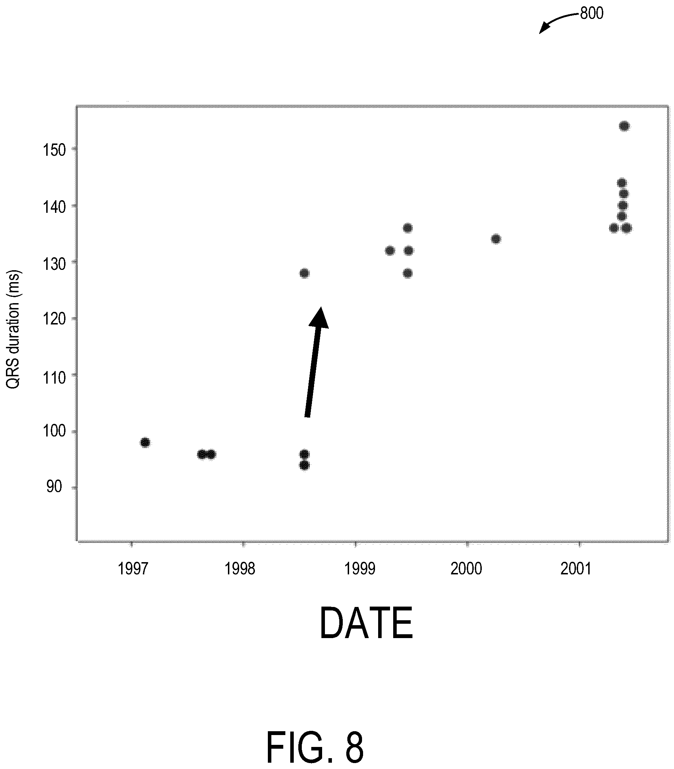

In contrast, a graph 800 depicting QRS duration over time in a patient with LBBB is shown in FIG. 8. For example, a patient may undergo multiple ECGs over a period of multiple years. As shown in graph 800, in LBBB, rather than a gradual increase, QRS duration may sharply and suddenly increase.

Thus, a most recently acquired ECG may include a QRS duration of 140, which could indicate either LVH or LBBB on its own. However, with the inclusion of longitudinal ECG data, QRS duration in previous ECGs may be considered. In this way, a gradual increase in QRS duration may be represented in a defined longitudinal feature, such as QRSDD, in patients with LVH while a sudden increase in QRS duration may be represented in the defined longitudinal feature in patients with LBBB. In this way, inclusion of longitudinal data, construction of longitudinal features, and extraction of longitudinal criteria may allow for more accurate diagnosis determine. Additionally, inclusion of longitudinal data in diagnosis determination by the ECG analysis system may reduce time spent by a provider in manually comparing ECGs to distinguish between diagnoses.

Turning to FIG. 6, a flowchart illustrating a method 600 for extracting criteria for determining a diagnosis by an ECG analysis model, such as a rules-based ECG analysis model. The method 600 may be executed by one or more processors (e.g., processor 220) according to instructions stored in non-transitory memory (e.g., memory 230). It should be appreciated that the method 600 herein described is exemplary in nature, and other methods of extracting criteria for determining a diagnosis by an ECG analysis model, such as a method that does not use an AI model.

At 602, method 600 includes receiving training data for training an AI model to extract criteria for determining a diagnosis by the ECG analysis model. As an example, a platform, such as platform 118 of the ECG analysis system 100 of FIG. 1, may receive training data for training the AI model, such as the AI model 120 of FIG. 1.

As an example, when the AI model is to be trained for single-ECG interpretation, the training data may include single ECGs as inputs and ECG criteria as targets. For example, the ECG may be a waveform acquired via the ECG device 110. Additionally or alternatively, the inputs of the training data may include a set of features for the ECG. For example, the set of features may include an amplitude of a P wave, a duration of the P wave, an amplitude of a Q wave, a duration of the Q wave, an amplitude of an R wave, a duration of the R wave, a PR interval, an amplitude of an S wave, a duration of the S wave, a duration of the QRS complex, an amplitude of a T wave, a duration of the T wave, a QT interval, or the like. Additionally or alternatively, the set of features may be defined by the data from a single lead (e.g., single lead specific P wave amplitude or P wave duration), or by data from multiple leads (e.g., global QT duration from earliest Q onset to latest T offset across all leads, or QT dispersion, which is a difference between shortest and longest single lead QT interval measurement across all leads), or by data from spatial relationships across multiple leads (e.g., spatial QRS-T angle or ventricular gradient).

As another example, when the AI model is to be trained for longitudinal ECG interpretation, the inputs of the training data may include multiple ECGs. For example the ECGs may be waveforms acquired via the ECG device 110, stored in the one or more medical data repositories, and retrieved by the platform 118. Additionally or alternatively, the inputs of the training data may include a set of longitudinal features of the ECGs. For example, the set of longitudinal features may include trend features for the aforementioned features, including a difference normalized to time (e.g., difference from a current ECG to an immediately previous ECG, and all its variations), the difference of the measurement from its mean of all ECGs of the patient normalized by standard deviation (e.g., measures of pull), and/or measure of current trend, which searches for the longest period over which a running trend can be defined (e.g., variation of normalized difference). Examples of longitudinal features include QRSDD and averaged or normalized QRSDD for a certain time period, as described above..

Other example longitudinal features to enhance confidence for an LBBB diagnosis include: 1) difference in QRS area measurements in leads V1 and V2 compared to a baseline or average for the patient; and 2) difference of T-wave amplitude/area and ST segment parameters in leads V5 and V6 compared to a baseline or average for the patient. When difference in QRS area measurements in leads V1 and V2 compared to a baseline or average for the patient becomes significantly negative (e.g., statistically significant, or more negative than a threshold amount), they indicate the development of deep and broad S waves in leads V1 and V2, which are characteristic of LBBB and abnormal for the patient (compared to their baseline). When difference in T-wave amplitude or area and SET segment parameters become significantly negative, they indicate the depression of ST segment and T-wave inversion in the lateral leads, which is characteristic of LBBB and abnormal for the patient (compared to their baseline).

Similar longitudinal parameters on ST segments, T-waves, and Q-waves that are abnormal for the patient can identify myocardial infarction with higher confidence than single-ECG criteria or parameters. More generally, a "score" parameter may be synthesized from a single ECG for a condition like the presence of structural heart disease. The increase of such a score parameter over time, as indicated by longitudinal features, can indicate the evolution of an underlying condition with higher confidence than just the single-ECG value of the score parameter.

Targets of the training data may include sets of ECG criteria. When the AI model is to be trained for single-ECG criteria extraction, the ECG criteria may be single-ECG criteria. When the AI model is to be trained for longitudinal criteria extraction, the ECG criteria may be longitudinal criteria. ECG criteria may comprise combinations of one or more ECG features that correspond to a diagnosis. For example, an input ECG may include a first feature and a second feature and a target criteria may be the combination of the first feature and second feature that together correspond to a diagnosis. So, for example, the AI model may be trained to ingest an ECG, identify the first and second feature, and extract therefrom, the criteria of the combination of the first and second feature.

Thus, the training data may include a diagnosis of an inputted ECG (e.g., the current ECG, in longitudinal examples). For example, the diagnosis may include atrial-paced rhythm, ventricular-paced rhythm, atrial flutter, ectopic atrial tachycardia, sinus bradycardia, sinus tachycardia, junctional bradycardia, atrial fibrillation, LBBB, LVH, septal infarct, non-ST elevated myocardial infarction, ST elevated myocardial infarction, or the like. Further, the diagnostic interpretation result may be an interpretation result of the ECG determined by a physician. Additionally or alternatively, the diagnostic interpretation result may be a diagnosis by a physician using the ECG alone, or alternatively using another source of clinical information (e.g., high sensitivity troponin levels, cardiac echo measurements, or angiography findings), or a combination of ECG and non-ECG clinical information.

Further, in some examples, the training data may include modification information that identifies whether the diagnostic interpretation result was modified. For example, the modification information may identify whether a diagnosis of the diagnostic interpretation result determined by the ECG analysis model 114 was accepted, removed, or replaced. As another example, the modification information may identify whether a diagnosis that was not determined by the ECG analysis model 114 was added. The modification information may identify a replacement diagnosis in the event that the diagnosis was replaced. According to an embodiment, the platform 118 may determine the modification information based on a diagnostic interpretation result determined by the ECG analysis model 114 and a final diagnostic interpretation result determined by a physician after the physician reviews the diagnostic interpretation determined by the ECG analysis model 114. For example, the platform 118 may compare the diagnostic interpretation result determined by the ECG analysis model 114 and the final diagnostic interpretation result determined by a physician, and determine the modification based on the comparison.

Further, the training data may include patient information associated with the ECG. For example, the patient information may identify whether a specific diagnosis was present in a previous diagnostic interpretation result of the patient, identify whether a physician had previously modified a diagnosis interpretation result, identify demographic information of the patient, identify health conditions (e.g., prior diagnosis, comorbidities, etc.) of the patient, identify medications prescribed to the patient, identify previous procedures performed on the patient, identify previous diagnoses (e.g., resolved diagnoses) of the patient, and the like.

Further, the training data may include a classification target of the ECG. For example, the classification target may be a diagnosis of the ECG, a diagnostic interpretation of the ECG, modification information of the ECG, or the like. The classification target may be a diagnostic statement, like LBBB, or myocardial infarction, or atrial fibrillation. The classification target may also be a modification by an expert of the diagnostic statement given by a baseline algorithm. For example, when a baseline algorithm gives a diagnostic statement (e.g., a diagnosis) like LBBB or atrial fibrillation, and there is data from retrospective databases where an expert edited/removed that diagnostic statement, the target in these instances can be to identify errors in the criteria of the baseline algorithm to reduce the amount of expert modification.

At 604, method 600 includes training the AI model with the training data of ECG features (e.g., inputs) and diagnoses criteria (e.g., targets). For example, the platform 118 of the ECG analysis system 100 of FIG. 1 may train the AI model 120 based on the training data. Alternatively, a system or device other than the platform 118 may be used to generate and/or train the AI model 120. For example, a system or device may include instructions for generating the AI model 120, and/or instructions for training the AI model 120. The system or device may provide a resulting trained AI model 120 to the platform 118 for use.

In some examples, the AI model 120 may include a training phase, a deployment phase, and a monitoring phase. In the training phase, the platform 118 may receive and process training data to generate the trained AI model 120 for extracting the criteria for determining a diagnosis by the ECG analysis model. The training data may include a plurality of training datasets respectively including one or more of ECG(s), a set of features of the ECG(s), a diagnosis of the ECG, a diagnostic interpretation of the ECG, modification information, patient information associated with the ECG, a classification target of the ECG, and/or the like. Each training dataset of the plurality of training datasets may be associated with a particular ECG and a particular diagnostic interpretation result.

The training data may be generated, received, or otherwise obtained from internal and/or external resources. For example, the training data may be generated, received, or otherwise obtained from the ECG device 110, the ECG analysis device 112, the user device 122, and/or the database 124.

Generally, the AI model 120 may include a set of variables (e.g., nodes, neurons, filters, or the like) that are tuned (e.g., weighted, biased, or the like) to different values via the application of the training data. According to an embodiment, the training process may employ supervised, unsupervised, semi-supervised, and/or reinforcement learning processes to train the AI model 120. According to an embodiment, a portion of the training data may be withheld during training and/or used to validate the trained AI model 120.

For supervised learning processes, the training data may include labels or scores that may facilitate the training process by providing a ground truth. For example, the labels or scores may indicate a classification target. Training may proceed by feeding a training dataset into the AI model 120. The AI model 120 may have variables set at initialized values (e.g., at random, based on Gaussian noise, based on pre-trained values, or the like). The AI model 120 may generate an output. The output may be compared with the corresponding label or score (e.g., the ground truth), which may then be back-propagated through the AI model 120 to adjust the values of the variables. This process may be repeated for a plurality of samples at least until a determined loss or error is below a predefined threshold. According to an embodiment, some of the training data may be withheld and used to further validate or test the trained AI model 120.

For unsupervised learning processes, the training data may not include pre-assigned labels or scores to aid the learning process. Instead, unsupervised learning processes may include clustering, classification, or the like, to identify naturally occurring patterns in the training data. As an example, training data may be clustered into groups based on identified similarities and/or patterns. K-means clustering or K-Nearest Neighbors may also be used, which may be supervised or unsupervised. Combinations of K-Nearest Neighbors and an unsupervised cluster technique may also be used. For semi-supervised learning, a combination of training data with pre-assigned labels or scores and training data without pre-assigned labels or scores may be used to train the AI model 120.

When reinforcement learning is employed, an agent (e.g., an algorithm) may be trained to make a decision regarding whether a diagnostic interpretation should be modified from the training data through trial and error. For example, based on making a decision, the agent may then receive feedback (e.g., a positive reward if the prediction was above a predetermined threshold), adjust its next decision to maximize the reward, and repeat until a loss function is optimized.

After being trained, the trained AI model 120 may be stored and subsequently applied by the platform 118 during the deployment phase. For example, during the deployment phase, the trained AI model 120 executed by the platform 118 may receive input data, and generate output data. During a monitoring phase, monitoring data may be analyzed along with the output data and input data to determine an accuracy of the trained AI model 120. According to an embodiment, based on the analysis, the platform 118 may return to the training phase, where values of one or more variables of the AI model 120 may be adjusted to improve the accuracy of the AI model 120.

According to an embodiment, the AI model 120 may be a decision tree. In this case, the platform 118 may generate the decision tree using a training technique. For example, the training technique may include a random forest technique, a boosted trees technique, a bootstrap technique, a rotation forest technique, or the like. The AI model 120 may include a set of nodes. For example, the set of nodes may include a root node, one or more intermediate nodes, and leaf nodes. The platform 118 may generate the AI model 120 using an attribute selection measure. For example, the attribute selection measure may be information gain, a gain ratio, a Gini index, or the like. The platform 118 may generate the decision tree, and prune the decision tree using a pruning technique. For example, the pruning technique may be cost complexity pruning, reduced error pruning, or the like.

At 606, method 600 includes extracting criteria for determining a diagnosis by the ECG analysis model. For example, the platform 118 of FIG. 1 may extract, using the AI model 120, the criteria for determining the diagnosis by the ECG analysis model 114.

In some examples, the platform 118 may determine a decision branch of the AI model 120 that corresponds to a particular target classification. For example, the target classification may be a diagnosis of the ECG, a diagnostic interpretation result of the ECG, modification information of the ECG, or the like. The decision branch may include one or more nodes corresponding to respective criteria. For example, the decision branch may include a root node, one or more intermediate nodes, and a leaf node.

Further, in some examples, the platform 118 may determine a decision branch of the AI model 120 that includes one or more nodes associated with an attribute selection measure that satisfies a threshold. For example, the attribute selection measure may be information gain, a gain ratio, a Gini index, or the like. As a particular example, the platform 118 may determine a decision branch that includes a leaf node corresponding to a criterion having a Gini index that is less than a threshold. According to an embodiment, the platform 118 may determine a decision branch based on a metric of the decision path. For example, the metric may be an accuracy, a positive prediction value, a sensitivity, or the like. Additionally, or alternatively, the platform 118 may determine a decision branch based on a performance on generalization to external datasets.

According to one or more examples, the platform 118 may determine particular nodes of the one or more nodes to use for criteria extraction. The platform 118 may determine the particular nodes based on a feature selection measure. The feature selection measure may correspond to an importance of a feature associated with a criterion corresponding to the node. According to an embodiment, the platform 118 may fit another AI model 120 (e.g., a decision tree) to a dataset that is separate from the training dataset. In this way, the platform 118 may reduce the risk of overfitting, and increase the statistical power of the technique.

In some examples, the platform 118 may extract the criteria from the decision branch. For example, the platform 118 may extract the criteria that belong to the decision branch. Each criterion may include a respective feature and a respective threshold. In some examples, the platform 118 may determine a threshold of a criterion. For example, the platform 118 may determine a threshold based on an optimization technique, based on an input from the user device 122, or the like.

Further, the platform 118 may generate a rule including a set of criteria that were extracted. For example, the rule may include criteria from a particular decision branch. Alternatively, the rule may include criteria from different decision branches. The platform 118 may generate the rule using a permutation and/or combination of criteria. According to an embodiment, the platform 118 may determine a metric of the rule. For example, the metric may be an accuracy, a positive prediction value, a sensitivity, or the like. Further, the platform 118 may determine that the rule includes a metric that satisfies a threshold.

Further still, the platform 118 may generate the rule to include a score threshold. For example, the score threshold may, if satisfied, trigger the ECG analysis model 114 to determine a diagnosis. According to one or more embodiments, the platform 118 may determine the score threshold based on a performance metric. For example, the performance metric may be an F-score, a precision, a sensitivity, or the like. Each criterion of the rule may be associated with a particular score. The ECG analysis model 114 may determine an overall score based on the particular criterion that are satisfied. The ECG analysis model 114 may compare the overall score to the score threshold, and determine a diagnosis based on comparing the overall score to the score threshold. For example, if the overall score is greater than or equal to the score threshold, then the ECG analysis model 114 may determine a particular diagnosis. Alternatively, if the overall score is less than the score threshold, then the ECG analysis model 114 may not determine the particular diagnosis. According to an embodiment, the platform 118 may generate the rule to include one or more mandatory criteria. For example, the rule may include a mandatory criterion that must be satisfied in order for the ECG analysis model 114 to determine a diagnosis regardless of the overall score. In this way, different decision branches may be explored by demanding that some of the criteria are met rather than being added to a score, and by varying how demanding each criterion is. The different decision branches that should be met before giving a positive classification may be thought of as accessing different subgroups, and may be deployed in parallel. For example, when assessing a diagnosis of atrial fibrillation, the platform 118 may consider two different subgroups (e.g., a first subgroup with many P waves detected and a second subgroup with very few P waves detected). Both subgroups may correctly identify relevant targets, but they operate in different parameter regions.

In some cases, the platform 118 may receive input from the user device 122 corresponding to expert knowledge that is leveraged to fine-tune the score. The expert knowledge may include an examination of all ECGs that are elected by the procedure for editing to assess the effect of the ECG analysis model 114, which is possible based on the full transparency of the criteria. If the expert knowledge indicates that some criteria are not accurate, or were selected due to bias from errors in annotations or labels, then the platform 118 may remove the criteria. If more control is warranted to avoid spurious decisions, then the platform 118 may demand concomitant validation from complementary or reciprocal leads and features. This control and accessibility is possible based on the interpretability of the criteria, and is unparalleled in other models.

In some examples, the platform 118 may validate the rule based on training data. For example, the platform 118 may receive training data from the database 124, and validate the rule. Based on validating the rule, the platform 118 may update the ECG analysis model 114 to implement the rule.

At 608, method 600 includes updating the ECG analysis model to determining a diagnosis using the extracted criteria. For example, the platform 118 may update the ECG analysis model 114 based on the criteria. For example, the ECG analysis model 114 may be updated to include the longitudinal criteria that are extracted such that the analysis model 114 may be used to determine a diagnosis based on longitudinal criteria. The platform 118 may update the ECG analysis model such that the ECG analysis model 114 may determine diagnoses using the criteria. In this way, the ECG analysis model 114 may receive ECGs, and determine diagnoses of the ECGs using the criteria extracted by the AI model 120.

It should be appreciated that method 600 is herein provided as an example for how to extract criteria and other methods may be performed without departing from the scope of this disclosure.

Turning now to FIG. 9, an example user interface 900 is shown. The user interface 900 may be displayed on a user device (e.g., user device 122). The user device may be configured to access one or more data repositories, such as EMRs, ECG databases, and the like. The user device may thus display, based on inputs applied to it, various patient information, including ECG data. For example, the user interface 900 may display data of ECGs, including identified features of the ECGs.

The user interface 900 includes a list of available ECGs. The list of the user interface 900 is sorted in FIG. 9 by patient (e.g., by patient ID (PID)). Thus, a first patient 902 may be listed multiple times in the list, one entry for each ECG of the first patient 902. The user interface may also indicate dates of acquisition 904 for each of the ECGs of the first patient 902.

Additionally, relevant or selected single-ECG features and longitudinal features may be displayed within the user interface 900. For example, a single-ECG feature column 906 may be displayed. The single-ECG feature column 906 may display QRS duration, in one example. A first longitudinal feature column 908 and a second longitudinal feature column 910 may also be displayed. The first longitudinal feature column 908 may display average QRS duration for all available ECGs up to the corresponding ECG entry. The second longitudinal feature column 910 may display QRSDD. For each ECG entry within the list, values of the features may be displayed in the corresponding column. The features displayed within the user interface 900 may be those ingested by the ECG analysis model in order to determine a diagnosis.

The technical effect of the herein disclosed systems and methods is that longitudinal data of a patient may be considered when determining a diagnosis from an ECG. Leveraging longitudinal data for diagnosis, by generating longitudinal features, extracting longitudinal criteria, and determining a diagnosis based on satisfied longitudinal criteria, may allow for more accurate diagnosis as well as saving time for physicians in determining diagnoses and comparing ECGs in order to determine a diagnosis.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A method, comprising:
generating a bank of longitudinal electrocardiogram (ECG) features from a plurality of ECGs (306), wherein the plurality of ECGs comprise current ECGs and one or more previous ECGs for each of the current ECGs;
extracting longitudinal criteria from the plurality of ECGs (410);
updating an ECG analysis model with the longitudinal criteria (414);
deploying the ECG analysis model to determine a diagnosis for an ECG of a patient based on the longitudinal criteria (510), wherein one or more corresponding previous ECGs are available for the patient; and transmitting the diagnosis to a user device (514).

2. The method of claim 1, wherein generating the bank of longitudinal ECG features includes:
extracting criteria of a plurality of single ECG records (302);
identifying most relevant ECG parameters in each of the plurality of single ECG records based on the extracted criteria (304); and
generating longitudinal ECG features for each of the identified most relevant ECG parameters (306).

3. The method of claim 2, wherein each of the plurality of single ECG records have one or more corresponding historical ECGs and wherein the longitudinal ECG features are generated based on the identified most relevant ECG parameters within each of the plurality of single ECGs and respective corresponding historical ECGs (304, 306).

4. The method of claim 2, wherein the criteria of the plurality of single ECG records are extracted using an AI model trained to extract criteria based on features identified within the plurality of single ECG records (302, 604).

5. The method of claim 1, further comprising comparing a performance of the criteria extracted based on the longitudinal criteria to a performance of criteria extracted from the current ECG alone (508).

6. The method of claim 5, wherein the diagnosis is determined based on the longitudinal criteria in response to determination that the performance of the criteria extracted based on the longitudinal criteria is greater than the performance of criteria extracted from the current ECG alone (510).

7. The method of claim 1, wherein the longitudinal criteria are extracted using an AI model trained to extract longitudinal features based on identified longitudinal features in the plurality of ECGs (410, 604).

8. The method of claim 7, wherein the AI model is a decision tree and the longitudinal criteria are extracted by the AI model based on a decision branch of the decision tree (604).

9. A device, comprising:
a memory (230) configured to store instructions; and
one or more processors (220) configured to, based on the instructions stored in the memory:
during a development phase of an ECG analysis model, receive a plurality of electrocardiograms (ECGs) with known diagnoses, wherein one or more of the plurality of ECGs have one or more ECGs previously acquired for the same patient (402);
determine one or more longitudinal features of the plurality of ECGs (408);
extract one or more longitudinal criteria based on the one or more longitudinal features (410);
update the ECG analysis model based on the one or more longitudinal criteria (414);
during a deployment phase of the ECG analysis model, determine a diagnosis of a newly acquired ECG using the one or more longitudinal criteria extracted from the plurality of ECGs (510); and
transmit the diagnosis to a user device communicatively coupled to the device (514).

10. The device of claim 9, wherein the one or more processors are further configured to obtain medical record data for a plurality of patients from one or more medical data repositories (402) and identify, from the plurality of patients, a subset of patients, including the patient, with records including more than one ECG (404).

11. The device of claim 9, wherein the one or more longitudinal features determined of the plurality of ECGs are a subset of a bank of longitudinal features generated by:
extracting criteria of a plurality of single ECG records (302);
identifying most relevant ECG parameters in each of the plurality of single ECG records based on the extracted criteria (304); and
generating longitudinal ECG features for each of the identified most relevant ECG parameters (306).

12. The device of claim 9, wherein the longitudinal features indicate historical data of the patient.

13. The device of claim 9, wherein the one or more longitudinal criteria of the plurality of ECGs are extracted using an AI model trained to extract longitudinal criteria based on the longitudinal features identified within the plurality of ECGs (604).

14. The device of claim 9, wherein the one or more processors are further configured to determine a performance of the longitudinal criteria. (412)

15. The device of claim 14, wherein the one or more processors are further configured to compare the performance of the longitudinal criteria to a performance of single-ECG criteria (508).
